# EUROPEAN PATENT APPLICATION

(11) **EP 2 599 766 A1**
(43) Date of publication of application: **05.06.2013**
(21) Application number: 12194160.3
(22) Date of filing: 26.11.2012
(51) Int. Cl.: C07C 51/41, C07C 57/03, C07C 57/12, C10M 129/60, C10M 159/20, C08K 3/24

(54) **Overbased calcium carboxylate/carbonate compositions**

(30) Priority: 29.11.2011 IT VA20110032
(71) Applicant: Lamberti SPA, 21041 Albizzate (VA) (IT)
(72) Inventor: Romagnano, Stefano, 20020 Lainate (MI) (IT); Floridi, Giovanni, 28100 Novara (IT); Li Bassi, Giuseppe, 21026 Gavirate (VA) (IT)
(74) Representative: Giaroni, Paola

(57) **Abstract**

This invention relates to overbased calcium carboxylate/carbonate compositions to be used in formulations for the stabilization of halogen containing polymers such as polyvinyl chloride (PVC) resins.

In addition this invention relates to a process for the preparation of overbased calcium salts comprising the use of plasticizers as solvent and of linear aliphatic carboxylic acids.

## Description

### TECHNICAL FIELD

This invention relates to overbased calcium carboxylate/carbonate compositions to be used in formulations for the stabilization of halogen containing polymers such as polyvinyl chloride (PVC) resins.

In addition this invention relates to a process for the preparation of overbased calcium salts comprising the use of plasticizers as solvent and of unsaturated linear aliphatic carboxylic acids.

### PRIOR ART

Several patents (i.e. US 3,766,067, US 5,830,832, US 6,348,164, US 2004/0162372, US 2004/0053795) describe the preparation of overbased calcium fatty carboxylic salts, by reaction of calcium oxide or hydroxide with aliphatic acids and successive carbonation with gaseous CO₂. The use of overbased metal salts in halogen-containing organic polymers is described in any of the above mentioned patents as well as in US 4,159,973, US 4,252,698 and US 3,194,823.

The conventional processes for the preparation of calcium overbased salts entails the use of both a promoter, generally based on alcohols and/or phenolic derivatives, and a catalyst, such as Zn octoate or low molecular weight aliphatic acids. In most cases the reaction solvents are organic alkyl or alkylaryl or aralkyl compounds, like white spirit. Such inert solvents only act as diluents and they have not any useful influence on the stabilized halogen containing polymers (resins). On the contrary, they contain volatile fractions (VOC) that can be released by the resins or the manufactured products when they are exposed to moderate heat, typically at temperature ranging from about 60° to about 130°C, producing an undesired effect commonly called "fogging". Fogging is a very critical problem for applications of stabilized halogen containing resins in the automotive, flooring or food-packaging sectors.

In addition, applications such as the manufacture of food contact packaging materials or medical articles require the use of stabilizer formulations which contain ingredients that are approved for the use in non-toxic halogen containing polymer articles. This precludes the use, for example, of phenolic compounds and solvents that are not enclosed in the positive list.

In order to reduce the release of volatile components when heated, US 6,835,328 reports the use as solvents of paraffinic oils having flash point higher than about 170°C.

To avoid the presence of solvents that act as contaminant and limit the use of stabilized halogen containing resins, WO 94/26686 describes the preparation of specific calcium superbase soaps in a plasticizer, in the presence of a catalyst. The carboxylic fatty acids used in WO 94/26686 consist of a mixture of saturated organic carboxylic acids containing from 7 to 13 carbon atoms and having a linear acid content of between 0 and 40% by weight, a content of acids which are branched on carbon 2 of between 0 and 20% by weight, and a content of acids which are mono- or poly-substituted on carbon 3 and/or on the carbons of higher rank, which is equal to or greater than 50% by weight. According to WO 94/26686 pure linear carboxylic acids are not suitable for the direct preparation of calcium overbased salts in solvent free mediums based on plasticizers, because they give very viscous products not suitable for subsequent filtrations and manipulations. Moreover, as reported in the Examples of GB 9309946 (the priority document of WO 94/26686), even the product obtained with the process of the invention using branched saturated carboxylic acid at C-3 and/or at carbons of higher rank, is a thick paste at room temperature, which makes its filtration impossible and its manipulation very difficult.

However, as it is well known by those skilled in the art, the presence of linear aliphatic carboxylic acids containing more than 10 carbon atoms, and particularly more than 14 carbon atoms, would be advantageous in thermal stabilizers for halogen containing polymer because the corresponding metal salts are more compatible with plasticized halogenated resins, due to their lipophilic characteristics. Stabilizing formulations containing the calcium overbased salts of these linear aliphatic carboxylic acid are also self-lubricating. In particular they avoid or largely reduce the problem of plate-out, i.e. the segregation of part of the metal salt from the stabilized halogen containing polymers, in particular during the calendering process. Accordingly, there is a need for VOC-free process for the preparation of overbased Ca salts of linear aliphatic carboxylic acids in plasticizers as solvent.

We have surprisingly found that it is possible to directly prepare flowable overbased calcium carboxylate/carbonate compositions in a plasticizer by reaction of calcium hydroxide or oxide with the linear aliphatic carboxylic acid and CO₂ in the plasticizer that acts as reaction medium, provided that the linear carboxylic acids are unsaturated or mainly unsaturated. Such preparation requires the use of unsaturated linear aliphatic carboxylic acids or of mixture of saturated and unsaturated linear aliphatic carboxylic acids , the mixture having a iodine number higher than 50.

An advantageous characteristic of the above overbased calcium carboxylates/carbonates prepared by unsaturated aliphatic linear carboxylic acids in a plasticizer is that they are obtained without the need to make the reaction in a relatively low boiling point solvent, to distillate off the solvent and to dissolve the solid overbased calcium carboxylate/carbonate in the plasticizer. Moreover we found that there is no need of catalysts to carry out the reaction. Only the use of a proper amount of promoters is required, acting as phase-transfer agents, preferably of the class of aliphatic hydroxy containing compounds, which avoids the presence of undesirable phenol or phenolic derivatives.

With the definition "overbased" we mean that, on an equivalents basis, the total amount of all of calcium present in the compositions of the invention exceeds the total amount of linear aliphatic carboxylic acids present.

### DESCRIPTION OF THE INVENTION

It is therefore an object of the present invention a process for the preparation of overbased calcium carboxylate/carbonate compositions comprising the step of :
a) reacting calcium hydroxide or oxide with one or more C₁₀-C₂₂ linear unsaturated aliphatic carboxylic acids or with mixtures of C₁₀-C₂₂ linear aliphatic carboxylic acids having a iodine number higher than 50, preferably higher than 70, with an equivalent ratio of calcium ions to carboxylic acids greater than 1, in the presence of a plasticizer as solvent and of an aliphatic hydroxy containing compound as promoter to obtain overbased calcium carboxylates;
b) carbonating the overbased calcium carboxylates of step a) with gaseous carbon dioxide to produce overbased calcium carboxylates/carbonates;
c) distilling off water and volatile components under vacuum to obtain flowable liquid compositions of overbased calcium carboxylates/carbonates;
d) filtering off unwanted impurities or by-products, obtaining a clear, stable, haze-free liquid overbased calcium carboxylate/carbonate compositions.

Another object of the invention are the overbased calcium carboxylate/carbonate compositions obtained with the above described process.

Formulations for the stabilization of halogen containing polymers comprising said overbased calcium carboxylate/carbonate compositions are a further object of the invention.

### DETAILED DESCRIPTION OF THE INVENTION

In step a) the equivalent ratio of calcium ions to linear aliphatic carboxylic acid is between 1 and 6 and preferably between 2 and 4.

The linear aliphatic carboxylic acids of step a) comprise more than 90 % by weight, preferably more than 95 % by weight, of linear (unbranched) carboxylic acids.

C₁₀-C₂₂ linear unsaturated aliphatic carboxylic acids suitable for the process of the present invention include both unsaturated and polyunsaturated linear aliphatic carboxylic acids with from 10 to 22 carbon atoms, preferably with from 14 to 22 carbon atoms. Examples of these acids are palmitoleic acid, oleic acid, linoleic acid, linolenic acid, arachidonic acid.

Mixtures of C₁₀-C₂₂ linear aliphatic carboxylic acids utilizable comprise the above mentioned linear unsaturated aliphatic carboxylic acids and may also comprise one or more linear saturated aliphatic carboxylic acids, such as n-decanoic acid, n-undecanoic acid, pentadecanoic acid, palmitic acid, stearic acid, behenic acid, and the like, provided that the total iodine number of the mixture, which is an index of its unsaturation content, exceed 50.

Suitable mixtures are also the mixtures of carboxylic acid derived from natural oils such as olein, soy oil, canola oil and tall oil.

Among the mixtures of linear aliphatic carboxylic acids having a iodine number higher than 50, olein and tall oil fatty acids are preferred.

The iodine number is a measure of the unsaturation of fat, oil and their derivatives and is expressed in the terms of number of centigrams of iodine absorbed per grams of sample. It can be determined using the standard method ASTM D5554-95.

Plasticizers are additives that increase the plasticity or fluidity of PVC, mainly by significantly lowering its glass transition temperature.

Plasticizers suitable to be used as solvent in the present process are those commonly used in the field such as alkyl phthalates, alkyl terephthalates, cyclohexane dicarboxylic acid alkyl esters, alkyl adipates, alkyl trimellitates, polymeric plasticizers and mixture thereof.

Preferably, the plasticizers are PVC plasticizers selected among alkyl phthalates, alkyl terephthalates, cyclohexane dicarboxylic acid alkyl esters, alkyl adipates, alkyl trimellitates.

Dioctyl phthalate (DOP), diisononyl phthalate (DINP), diisodecyl phthalate (DIDP), diisononyl cyclohexan dicarboxylate (DINCH), dioctyl terephthalate (DOTP), bis-2-ethylhexyl terephthalate (BEHTP) and dioctyl adipate are preferred. DOTP, DINCH, DIDP, BEHTP are the most preferred plasticizers. The amount of plasticizer utilized in the preparation of the overbased calcium carboxylate/carbonate compositions ranges from 30 to 60 % by weight of the reaction mass, preferably from 45 to 55 %.

The preparation of the overbased metal carboxylates require the use of promoters which act as phase-transfer agents. In the present invention the promoter is chosen in the class of aliphatic hydroxy containing compounds. Suitable promoters include C₁-C₈ alcohols, glycols, glycol ethers and mixture thereof. Examples of these compounds include, but are not limited to, methanol, ethanol, propanol, i-propanol, n-butanol, i-butanol, tert-butanol, 2-ethylhexanol, ethylene glycol, diethylene glycol, triethylene glycol, propylene glycol, dipropylene glycol, tripropylene glycol, glycolethers such as Dowanol® compounds. Dipropylene glycol metyl ether (Dowanol® DPM) and i-butanol are the preferred aliphatic hydroxy containing compounds. The promoters are used in a proportion of 1 to 25 % by weight of the total reaction mass, and preferably in a proportion of 7 to 12% by weight.

As noted above, the process of the present disclosure is hydrocarbon-free; since no aliphatic or aromatic solvents, and in particular no low volatile compounds (VOCs), are added to the reaction mass.

After the reaction of the linear aliphatic carboxylic acids with calcium hydroxide is completed, the residual basic groups are carbonated (step b) by introducing gaseous carbon dioxide below the surface of the reaction mixture. The process of carbonation, which is a part of the process of obtaining the calcium carboxylate/carbonate, is well known to those skilled in the art. The amount of carbon dioxide gas used depends upon the amount of residual basic groups in the reaction mixture, however it is important to reach the completion of the carbonation process (no residual basic groups). The completion of the reaction can be controlled, for example, by IR monitoring the disappearance of the 3642 cm⁻¹ band of the OH stretching of calcium hydroxide.

The reaction steps a) and b) are carried out under stirring at temperature between 35 and 100 °C, preferably between 50 and 80 °C, under nitrogen atmosphere.

Water and possibly part of the promoter are removed by distillation under vacuum to obtain a flowable liquid which has a hazy appearance to the presence of unwanted volatile impurities or by-products. After the distillation the residual water content is usually below 1% by weight.

Filtration using filtration aids removes undesirable impurities including silica, iron oxide and other metal species, unreacted calcium hydroxide, calcium carbonate, which may contribute to lack of stability, and allows to obtain a stable haze-free liquid.

The resulting overbased calcium carboxylate/carbonate compositions comprise from 5 to 10% by weight of calcium ions; from 30 to 60 % by weight of plasticizer, preferably from 45 to 55 % by weight; from 1 to 25% by weight of promoter, preferably from 1 to 12% by weight. The equivalent ratio between calcium ions and linear aliphatic carboxylic acids is between 1 and 6 and preferably between 2 and 4.

A number of benefits are obtained by the process of the present invention. A product that does not contain aliphatic and aromatic hydrocarbon as solvents, that contaminate the formulation of stabilized halogen containing polymers (resins), and that contains lubricating components, that avoid plate-out, is achieved.

The overbased compositions so obtained minimize the fogging when the manufactured products are exposed to moderate heat, ranging from about 60 to about 130°C. Moreover they are free of phenol and phenolic derivatives reducing the environmental concern and avoiding a source of color development and with a proper selection of plasticizers they can be based on components approved for non-toxic applications.

The formulations for the stabilization of halogen containing polymers, in addition to the overbased calcium carboxylate/carbonate compositions described above, usually contain, some additional ingredients such as organic zinc salts and may also contain other ingredients such as solvents, epoxides such as epoxidized soybean oil or epoxidized linseed oil, β-diketones, organic phosphites, antioxidants, radical scavengers, optical brighteners, light stabilizers, perchlorates, fillers, plasticizers, impact modifiers, pigments and mixtures thereof. Such intermediates are conventionally employed in liquid stabilizer formulations and are readily recognized by those skilled in the art. Examples of halogen containing polymers that can be stabilized with the formulations of the invention are homo- or co-polymers derived from vinyl chloride (PVC) and/or from vinylidene chloride; vinyl resin containing vinyl chloride units in their structure, such as co-polymer of vinyl chloride and vinyl esters of aliphatic acid, in particular vinyl acetate; co-polymer of vinyl chloride with esters of acrylic and methacrylic acid and acrylonitrile; co-polymer of vinyl chloride with dienes and unsaturated dicarboxylic acids or their anhydrides, such as vinyl chloride with diethylmaleate, diethyl fumarate or maleic anhydride; chlorinated polymers obtained by chlorination after the polymerization; co-polymer of vinyl chloride or vinylidene chloride with unsaturated aldehydes and/or ketones such as acrolein, crotonaldehyde, vinylmethyl ketone, vinylmethyl ether, vinylisobutyl ketone and similar products; polymers of vinyl chloroacetate and dichlorovinyl ether; chlorinated polymers of vinyl acetate; chlorinated polymers of the esters of acrylic acid and/or alpha-replaced acrylic acids; chlorinated styrene polymers like dichlorostyrene; chlorinated ethylene polymers; post-chlorinated chlorobutadiene polymers and co-polymer with vinyl chloride; chlorinated natural and synthetic rubbers; mixtures of the above mentioned polymers, also with other polymerizable compounds. Within this invention, PVC also indicates co-polymers of vinyl chloride with other polymerizable compounds such as acrylonitrile, vinylacetate or ABS. These polymers can be in suspension, emulsion or mass.

The following Examples illustrate the preparation of the calcium overbased salts in accordance with the process of the present invention, but these examples are not considered to limit the scope of this invention. Unless otherwise indicated in the following examples and elsewhere in the specification and claims, all parts and percentages are by weight and all temperatures are in degrees Celsius.

### EXAMPLES

### Example 1

A 1000 ml four neck round bottom flask was charged with 254.1 g of DOTP, 15.8 g of Dowanol DPM and 38.6 g of i-butanol. The four neck round bottom flask was fitted with a nitrogen inlet, a thermometer, a distillation unit and a mechanical stirrer. The temperature was raised to 40 °C and portions of calcium hydroxide were added under stirring, making sure that all calcium hydroxide was mixed and did not clump up or stick to the bottom of the reactor. The total charge of calcium hydroxide was 59.2 g. Then the mixture was heated up to 70°C. When the temperature was stabilized, 180 g of olein with a iodine number of 98 cgI₂/g were added to the reaction mass. When the reaction between calcium hydroxide and olein was completed, the carbonation was started flowing CO₂ (flow rate 0,3 l/min) through the reaction mixture, monitoring the reaction by IR spectroscopy. When the IR peak at 3642 cm⁻¹ (OH stretching of calcium hydroxide) disappeared, the CO₂ addition was discontinued. The reaction mixture was then heated to about 140°C and water and i-butanol were distilled under vacuum. A total of 66 g of distillate was removed. The resulting reaction mixture was stirred and added with 5 g of filtration aid. Then the mixture was filtered by suction yielding 500 g of flowable pale yellow clear liquid which remained clear and stable upon cooling at room temperature. The content of calcium ions in the final product was 6.23 %.

### Example 2

The preparation was carried out according to Example 1, using 254.1 g of DIDP instead of DOTP. After filtration, 495 g of flowable yellowish to brownish clear liquid was obtained. The liquid remained clear and stable upon cooling at room temperature. The content of calcium ions in the final product was 6.33%.

### Example 3

The preparation was carried out according to Example 1, using 254.1 g of DINCH instead of DOTP. After filtration, 497 g of flowable yellowish to brownish clear liquid was obtained. The liquid remained clear and stable upon cooling at room temperature. The content of calcium ions in the final product was 6.29%.

### Example 4

The preparation was carried out according to Example 1, using 280.3 g of DIDP instead of DOTP and 153.8 g of a mixture of linear fatty acids with iodine number 52. At the end of the reaction between the fatty acids and calcium hydroxide, the reaction mass was very viscous. At the beginning of the carbonation (flow rate of CO₂ 0.3 l/min) the reaction mass is still very viscous; after 30 min of carbonation the reaction mass started to fluidize and became more fluid as the carbonation proceded. At the end of the carbonation the reaction mixture was heated to about 140°C to distill under vacuum water and i-butanol. The obtained reaction mass was fluid and suitable for filtration.

### Example 5

The preparation was carried out according to Example 1, using 252.5 g of DIDP instead of DOTP and 181.6 g of stearic acid instead of 180 g of olein. When the salification between calcium hydroxide and stearic acid was completed, an increase in the viscosity of the reaction mass was observed, until total solidification. The carbonation step was not performed.

### Example 6

The preparation was carried out according to Example 1, using 306.25 g of DIDP instead of DOTP and 127.85 g of lauric acid. At the end of the reaction of the lauric acid with calcium hydroxide, the reaction mass was very viscous with some solid inside. At the beginning of the carbonation (CO₂ flow rate 0,3 l/min) the viscosity improved a little, but after 30' of carbonation the reaction mass became a compact paste.

## Claims

1. Process for the preparation of overbased calcium carboxylate/carbonate compositions comprising the step of :
a. reacting calcium hydroxide or oxide with one or more C₁₀-C₂₂ linear unsaturated aliphatic carboxylic acids or with mixtures of C₁₀-C₂₂ linear aliphatic carboxylic acids having a iodine number higher than 50, with an equivalent ratio of calcium ions to carboxylic acids greater than 1, in the presence of a plasticizer as solvent and of a aliphatic hydroxy containing compound as promoter to obtain overbased calcium carboxylates;
b. carbonating the overbased calcium carboxylates of step a) with gaseous carbon dioxide to produce overbased calcium carboxylates/carbonates;
c. distilling off water and possible volatile components under vacuum obtaining a flowable liquid composition of overbased calcium carboxylates/carbonates;
d. filtering off unwanted impurities and/or by-products, obtaining a clear, stable, haze-free liquid overbased calcium carboxylate/carbonate compositions.

2. The process of Claim 1) wherein in step a) said mixtures of C₁₀-C₂₂ linear aliphatic carboxylic acids have a iodine number higher than 70.

3. The process of Claim 2) wherein said mixtures of C₁₀-C₂₂ linear aliphatic carboxylic acids are olein and tall oil fatty acids.

4. The process of Claim 1) wherein in step a) the equivalent ratio between calcium ions and linear aliphatic carboxylic acid is comprised between 1 and 6.

5. The process of Claim 1) wherein said plasticizer is chosen in the group consisting of alkyl phthalates, alkyl terephthalates, cyclohexane dicarboxylic acid alkyl esters, alkyl adipates, polymeric plasticizers, alkyl trimellitates and mixture thereof.

6. The process of Claim 5) wherein said plasticizer is chosen in the group consisting of dioctyl phthalate, diisononyl cyclohexan dicarboxylate, diisodecyl phthalate and bis-2-ethylhexyl terephthalate.

7. The process of Claim 1) wherein said aliphatic hydroxy containing compound is chosen in the group consisting of C₁-C₈ alcohols, glycols, glycol ethers and mixture thereof.

8. The process of Claim 7) wherein said aliphatic hydroxy containing compound is choasen among dipropylene glycol metyl ether and i-butanol.

9. Overbased calcium carboxylate/carbonate compositions obtained with the process of Claim 1) which comprise from 5% to 10% by weight of calcium ions; from 30 to 60 % by weight of plasticizer; from 1 to 25% by weight of promoter and wherein the equivalent ratio between calcium ions and linear aliphatic carboxylic acids is between 1 and 6.

10. Formulations for the stabilization of halogen containing polymers comprising the overbased calcium carboxylate/carbonate compositions of Claim 9).
